# EUROPEAN PATENT SPECIFICATION

(11) **EP 3 342 855 B1**
(45) Date of publication and mention of the grant of the patent: **23.09.2020**
(21) Application number: 17208723.1
(22) Date of filing: 20.12.2017
(51) Int. Cl.: C12N 5/00

(54) **CELL COMPOSITION DEPLETED FROM TCRAB AND CD45RA POSITIVE CELLS**
ZELLZUSAMMENSETZUNG, DIE AUS TCRAB- UND CD45RA-POSITIVEN ZELLEN VERARMT IST
COMPOSITION CELLULAIRE APPAUVRIE EN CELLULES TCRAB ET CD45RA POSITIVES

(30) Priority: 27.12.2016 EP 16206909
(43) Date of publication of application: 04.07.2018
(73) Proprietor: Miltenyi Biotec B.V. & Co. KG, 51429 Bergisch Gladbach (DE)
(72) Inventor: HUPPERT, Volker, 51515 Kürten (DE); MILTENYI, Stefan, 51429 Bergisch Gladbach (DE); DZIONEK, Julia, 51491 Overath (DE); LEUNG, Wing, Cambridge, MA 02140 (US)
(74) Representative: Kisters, Michael Marcus

(56) References cited:
- WO-A1-2012/123590
- NICHOLAS BRODSZKI ET AL: "Novel treatment of severe combined immunodeficiency utilizing ex-vivo T-cell depleted haploidentical hematopoietic stem cell transplantation and CD45RA+ depleted donor lymphocyte infusions", ORPHANET JOURNAL OF RARE DISEASES, vol. 11, no. 1, 15 January 2016 (2016-01-15), XP055358284, DOI: 10.1186/s13023-016-0385-3
- RUPERT HANDGRETINGER: "Negative depletion of CD3+ and TcR[alpha][beta]+ T cells", CURRENT OPINION IN HEMATOLOGY, vol. 19, no. 6, 1 November 2012 (2012-11-01), pages 434-439, XP055358538, US ISSN: 1065-6251, DOI: 10.1097/MOH.0b013e3283582340
- A. BERTAINA ET AL: "HLA-haploidentical stem cell transplantation after removal of + T and B cells in children with nonmalignant disorders", BLOOD, vol. 124, no. 5, 28 May 2014 (2014-05-28), pages 822-826, XP055361457, & 53RD ANNUAL MEETING AND EXPOSITION OF THE AMERICAN-SOCIETY-OF-HEMATOLOGY (ASH); SAN DIEGO, CA, USA; DECEMBER 10 -13, 2011 ISSN: 0006-4971, DOI: 10.1182/blood-2014-03-563817
- P LANG ET AL: "Improved immune recovery after transplantation of TCR[alpha][beta]/CD19-depleted allografts from haploidentical donors in pediatric patients", BONE MARROW TRANSPLANTATION, vol. 50, 1 June 2015 (2015-06-01), pages S6-S10, XP055361458, GB ISSN: 0268-3369, DOI: 10.1038/bmt.2015.87
- TOUZOT FABIEN ET AL: "CD45RA depletion in HLA-mismatched allogeneic hematopoietic stem cell transplantation for primary combined immunodeficiency: A preliminary study", JOURNAL OF ALLERGY AND CLINICAL IMMUNOLOGY, ELSEVIER, AMSTERDAM, NL, vol. 135, no. 5, 3 October 2014 (2014-10-03), page 1303, XP029574861, ISSN: 0091-6749, DOI: 10.1016/J.JACI.2014.08.019
- D TESCHNER ET AL: "Depletion of naive T cells using clinical grade magnetic CD45RA beads: a new approach for GVHD prophylaxis", BONE MARROW TRANSPLANTATION, vol. 49, no. 1, 12 August 2013 (2013-08-12), pages 138-144, XP055358248, GB ISSN: 0268-3369, DOI: 10.1038/bmt.2013.114
- Anonymous: "CliniMACS CD45RA Depletion System | Reference list", , 1 March 2015 (2015-03-01), page 218, XP055358252, Retrieved from the Internet: URL:http://www.miltenyibiotec.com/~/media/ Images/Products/Import/0009700/IM0009781.a shx [retrieved on 2017-03-23]

## Description

### FIELD OF THE INVENTION

This invention relates to a method to provide a cell composition obtainable from bone marrow or blood, wherein the cell population is depleted of TCR alpha/beta positive cells and CD45RA positive cells.

### BACKGROUND

Stem cells transplantations are more and more utilized for the treatment of hematological, oncological, immunological and genetic diseases. Usually, stem cells are extracted from bone marrow or mobilized blood processed after leukapheresis.

Depending on the donor, stem cell transplantations still suffer from complications, originating from the reaction of the transplants against the recipients (Graft-versus-host-disease, GvHD or GvHR). Other common complications include failure of engraftment of the transplanted stem cells, the toxicity of the conditioning steps, or the infections under therapy due to a prolonged or incomplete immune reconstitution.

In order to avoid complications and to tailor the stem cell transplant to a given recipient, it is known to enrich and/or deplete certain cell subpopulations from the starting cell composition. In this regard, several depletion and/or enrichment strategies are described in the prior art. Especially, the enrichment of CD34+ cells and the depletion of CD3+ cells are known processes in this field.

In a another process disclosed by US20140308250A1, a cell population obtainable from bone marrow or blood is depleted of TCR alpha/beta positive cells and simultaneously of CD19 positive cells. The thus obtained cell population may be used for the reconstitution of hematopoietic system of a human after a stem cell or bone marrow transplantation. This cell population contains additional immune cells, e.g. Natural Killer cells, gamma/delta T cells, dendritic cells, monocytes. These cell population either have an anti-tumor and/or anti-virus effect or contribute to engraftment of the stem cells. However, the cell composition obtained by the process disclosed in US20140308250A1 does not contain pathogen specific T cells, which could address infection more efficiently than NK cells and gamma/delta T cells alone.

It is known to isolate virus specific T cells from a separate cell sample based on cytokine secretion upon virus antigen encounter. This method is expensive, time consuming and results in low amounts of cells limited to reactivity against antigens used for processing. Further approaches on specific T cell separation from a cell sample are disclosed in the following publications:
NICHOLAS BRODSZKI ET AL: "Novel treatment of severe combined immunodeficiency utilizing ex-vivo T-cell depleted haploidentical hematopoietic stem cell transplantation and CD45RA+ depleted donor lymphocyte infusions", ORPHANET JOURNAL OF RARE DISEASES, vol. 11, no. 1, 15 January 2016 (2016-01-15);
WO 2012/123590 A1;
RUPERT HANDGRETINGER: Negative depletion of CD3+ and TcR[alpha] [beta]+ T cells", CURRENT OPINION IN HEMATOLOGY, vol. 19, no. 6, 1 November 2012 (2012-11-01), pages 434-439;
A. BERTAINA ET AL: "HLA-haploidentical 1-12 stem cell transplantation after removal of + T and B cells in children with nonmalignant disorders", BLOOD, vol. 124, no. 5, 28 May 2014 (2014-05-28), pages 822-826;
P LANG ET AL: "Improved immune recovery after transplantation of TCR[alpha] [beta]/CD19-depleted allografts from haploidentical donors in pediatric patients", BONE MARROW TRANSPLANTATION, vol. 50, 1 June 2015 (2015-06-01), pages S6-S10;
TOUZOT FABIEN ET AL: "CD45RA depletion in HLA-mismatched allogeneic hematopoietic stem cell transplantation for primary combined immunodeficiency: A preliminary study", JOURNAL OF ALLERGY AND CLINICAL IMMUNOLOGY, ELSEVIER, AMSTERDAM, NL, vol. 135, no. 5, 3 October 2014 (2014-10-03), page 1303;
D TESCHNER ET AL: "Depletion of naive T cells using clinical grade magnetic CD45RA beads: a new approach for GVHD prophylaxis", BONE MARROW TRANSPLANTATION, vol. 49, no. 1, 12 August 2013 (2013-08-12)

Object of the invention was to provide a method that is suitable for the reconstruction of the hematopoietic system by depletion of TCR alpha/beta and CD45RA positive cells, but which still contains memory T cells, in addition to NK cells, TCRgamma/delta T cells, B cells and blood dendritic cells.

Whereas the primary application of the invention is in stem cell transplantation, the invention may also be used in other clinical settings including adoptive cellular therapy such as donor lymphocyte infusion for the treatment of infection, mixed chimerism, and cancer recurrence.

### SUMMARY

The first aspect of the present invention is a method for the preparation of a cell population from a sample originating from bone marrow or blood, comprising the steps a) dividing the sample into a first fraction containing 50 to 99 % of the cells of the sample and a second fraction containing 50 to 1 % of the cells of the sample, b) labeling the cells of the first fraction with a first marker against TCR alpha/beta, c) labeling the cells of the second fraction with a second marker against CD45RA, d) removing the labeled cells from the first and second fraction and combining the remaining cells to a cell population.

### BRIEF DESCRIPTION OF THE DRAWINGS

Various exemplary details are described with reference to the following figures, wherein:
Fig. 1 shows 1 shows the IFNγ-Production by CD4+ and CD8+ T cells for the leukapheresis, TCRab depleted fraction and mixture 95:5 (TCRab depleted : CD45RA depleted) minus the negative control for each sample.
Fig. 2 shows IFNγ-Production by CD4+ and CD8+ T cells for TCRab depleted fraction and mixture 95:5 (TCRab depleted : CD45RA depleted) minus the negative control for each sample

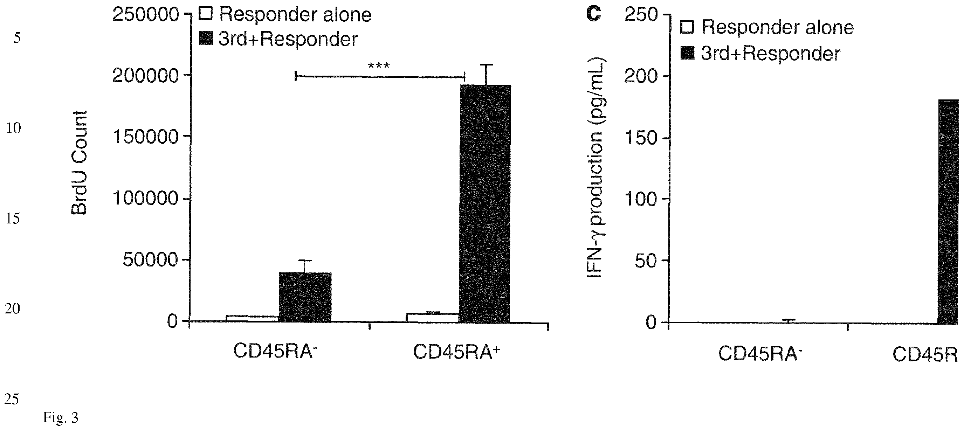

Fig. 3 shows BrdU count of cells and interferon gamma content of cell culture supernatants of CD45RA negative and CD45RA positive cells upon interaction with allogeneic cells.

### DETAILED DESCRIPTION

The method of the invention involves the preparation of a cell population from a sample originating from bone marrow or blood. The term "sample originating from bone marrow or blood" includes all cell populations obtainable from bone marrow, fractions thereof or pre-processed bone marrow, whole blood, fractions of blood, blood products or processed blood, cell preparations obtained by leukocyte apheresis (leukapheresis), venipuncture or bone marrow puncture. Preferably, the cell preparation is obtained from a healthy donor who was previously treated with stem cell mobilizing drugs in the setting of stem cell transplantation.

The term "depletion" refers to a reduction of the amount of certain cells from a cell population. The depletion may be based on the amount of cells defined by presence (or absence) of, for example, a cell surface marker, such as TCR alpha/beta or CD45RA) by at least two logarithmic steps, preferably by at least three logarithmic steps, particularly preferred by at least 4 logarithmic steps (e.g., 4.6 logarithmic steps), most preferred by at least four to five logarithmic steps.

The removal according to logarithmic steps is as follows: 1 log:90% removal of the unwanted cells, 2 log:99%, 3 log:99.9% and 4 log:99.99%. Methods for calculating the separation performance are known to a person of skill in the art and described, for example, in Bosio et al., Isolation and Enrichment of Stem Cells, Advances in Biochemical Engineering and Biotechnology, Springer Verlag Berlin Heidelberg, 2009.

The depletion process of the invention is performed using the cell surface marker TCR alpha/beta and the surface marker CD45RA. The depletion can be performed with any technique known in state of the art, e.g. panning, elutriation or magnetic cell separation. Preferred is a depletion using magnetic cell separation, for example with the CLINIMACS Plus or CLINIMACS Prodigy instrument, both obtainable from Miltenyi Biotec GmbH due to the high depletion efficiency.

In further variants, the invention refers to a method, in particular, an in vitro method for the preparation of a population of cells. The method comprises the following steps:
- providing a cell sample originating from bone marrow or blood of a donor (that is of a population that comprises, amongst others, TCR alpha/beta positive and CD45RA positive cells)
- splitting the cell sample into a first and second fraction
- depletion of TCR alpha/beta positive cells from the first cell population.
- depletion of CD45RA positive cells from the second population
- combining the TCR alpha/beta and CD45RA depleted products

Preferably, the depletion of TCR alpha/beta positive cells is performed using an antibody or antigen-binding fragment against TCR alpha/beta. On the basis of the protein or nucleotide sequences according to SEQ ID NOs 1 to 11 of the receptor TCR alpha/beta (see sequence protocols), an antibody or antigen fragment, or a derivative or conjugate thereof against TCR alpha/beta can be produced and used for the depletion of the TCR alpha/beta positive cells.

In a preferred embodiment, the method further comprises the following step: labeling cells expressing CD19 of the first fraction and/or the second fraction and/or the cell population with a third marker and removing the labeled cells.

### LABELLING

The first, second and/or third marker used in the process of the invention may comprise an antibody or an antigen-binding fragment against TCR alpha/beta and/or against CD45RA and/or CD19, respectively and a detection moiety.

The detection moiety of the markers may be the same or different and may be a fluorescence dye, a magnetic particle or a radioactive label.

CD45RA is a surface molecule on naive T cells and B cells. The term CD45RA positive cells refers to cells expressing the CD45RA molecule on the surface and to which an appropriate CD45RA -binding molecule, for example an antibody against CD45RA can specifically bind.

TCR alpha-beta is a surface molecule on T cells. The term "TCR alpha/beta positive cells" refers to a cell expressing the TCR alpha/beta molecule on the surface and to which an appropriate TCR alpha/beta-binding molecule, for example, an antibody can specifically bind.

The term "Antibody" refers to any monoclonal or polyclonal antibody of human or animal origin like be rat, rabbit, goat, horse or mice a to derivative of these antibodies that largely retains the binding capacity or the original antibody. Preferred derivatives of these antibodies are chimeric antibodies comprising, for example, chimeric antibodies of a variable region or the mouse or the rat and a human constant region. The term "antibody" comprises also bi-functional or bi-specific antibody and antibody constructs like Fvs (scFv) from single chain or antibody fusion proteins. The term "scFv" (single chain Fv Fragment) is known to a person skilled in the art and is preferred that the fragment is produced in a recombinant fashion.

Antibodies utilized in the present invention may be human or humanized. The term "humanized antibody" refers to an human antibody wherein at least one antibody binding site (complementary determining region, CDR), like for example, CDR3 and preferably all six CDRs were substituted by CDRs from a human antibody with the desired specificity. Optionally, the non-human constant region(s) was replaced by a constant region(s) of a human antibody. Methods for producing human antibodies are described for example in EP 0239400 A1 and WO 90/07861 A1.

The term antigen-binding fragment refers to a fragment of an antibody as defined above like for example separated light and heavy chains, Fab, ab/c, Fv, Fab' F(ab')2. An antigen-binding fragment can comprise a variable region of the light chain and a variable region of the heavy chain, not necessarily both together.

The detection moiety of the first, second and/or third marker may be a fluorescence dye, a magnetic particle or a radioactive label. Suitable detection moieties and processes to conjugate the detection moieties to the antibody or an antigen-binding fragments are well known to the person skilled in the art.

### DEPLETION

In the method of the invention, the cells of the sample are divided into a first fraction and second fraction. Preferable, the first fraction contains 60% to 99%, 70% to 99% 75 to 99%, 85 to 99 %, 90 to 99 % or 95 to 99 % of the cells of the sample, and the second fraction the respective range to add up to 100%.

After labelling the cells of the fractions as already described, the labeled cells from the first and second fraction are removed from the sample. Removal of the labeled cells is accomplished based on the nature of the detection moiety. In case the detection moiety is a fluorescence dye, removal in performed on fluorescence-based methods like FACS or using the TYTO instrument (Miltenyi Biotec GmbH). In case the detection moiety is a magnetic particle, magnetic cell sorting as known to the person skilled in the art is used. A preferred embodiment comprising magnetic cell sorting is described later.

Removal of the labeled cells may be accomplished in a first embodiment wherein in step d), first and second fraction are combined and the labeled cells are removed from the combined fraction. This strategy is preferred when the fractions contain detection moieties of a different nature like fluorescence dye and magnetic particle or if a more purer depletion is desired.

In a second embodiment of the invention, in step d), labeled cells are removed from the first and second fraction separately and the remaining cells of each fraction are combined. The second strategy is preferred when detection moieties having the same nature or even identical detection moieties are used and/or a faster processing is desired.

The process of the invention may be performed in several variants, for example:
variant A) deplete CD45RA cells from 10% cells of sample, then deplete TCRalpha/beta (or TCRalpha/beta and CD19) from 90% of sample, combine both depleted cell samples
variant B) deplete CD45RA from 10% of sample, then deplete TCRalpha/beta (or TCRalpha/beta and CD19) from 90% of sample, but retain both samples as two separate, individual products
variant C) label 10% of sample with CD45RA, then label 90% of sample with TCRalpha/beta-Biotin, combine resulting fractions, then label whole product with anti-Biotin Reagent and separate magnetically. Optionally perform wash steps between/after each labeling step
variant D) label 10% of sample with CD45RA, then label 90% of sample with TCRalpha/beta-Biotin, label with anti-Biotin Reagent, combine resulting fractions and separate magnetically. Optionally perform wash steps between/after each labeling step
variant E) magnetically depleting steps by single passage over column or two passages over column ("bulk depletion" + "sensitive depletion") (results in highest depletion efficiency)
variant F) magnetically deplete CD45RA part by a single passage over column, magnetically deplete TCRalpha/beta part by two passages
variant G) process sample and then rebuffer into a solution feasible for infusion
variant H) process sample with infusion solution (rather than CliniMACS buffer)

In all variants of removal of labeled cells alike, the remaining cells are combined into a cell population lacking at least a part of TCR ab and CD45RA positive cells. Preferably, the total number of TCRab and CD45RA double positive cells is less than 25 thousands per kilogram recipient weight or less than 0.1% of the total nucleated cell population, like less than 0.03 or even less than 0.005%.

For example, TCRalpha/beta and CD45RA double positive T cells should be depleted by more than 99.99% (4 log) to a final cell dose of less than 25,000 TCRab+/CD45RA+ cells per kg of bodyweight of the patient, i.e. to less than 2.5 million cells for a 100 kg patient. TCRalpha/beta positive / CD45RA negative memory T cells should be retained to a safe but immunological effective dose, i.e. 0.1 million to 100 million/kg, or 10E6 to 10E9 cells for a 100 kg patient. TCRalpha/beta negative cells (both CD45RA positive and negative cell populations) should be maintained (lowest possible depletion), e.g. to a total cell dose of about 2.4E9 gamma/delta T cells (i.e. in a range or 5 - 250 million/kg), or 24 million gamma/delta T cells for a 100 kg patients (i.e. in a range of 500E6 to 25E9).

### AUTOMATIC PROCESSING

In a preferred embodiment, the entire process is performed in an automated, closed system comprising storage containers for the cell sample, the labelling conjugates, washing buffer, waste and the desired cell population, one or more centrifuge chambers, detection and separation means appropriate for the detection moieties of the labelling conjugates and a tubing set for connecting purposes. The term "automated, closed system" refers to a system adapted to sterile usage i.e. air and fluid-tight closed with or without means for sterile pressure compensation and without the need of manual interaction. Such system is for example described in WO2009072003A2 or WO2009072006A2 and commercialized under the tradename CLINIMACS Plus or CLINIMACS Prodigy by Miltenyi Biotec GmbH.

Automated processing includes splitting of the starting material into a predefined split ratio for first and second fraction, determining the cell number in the sample, adding an appropriate amount of antibodies or cell separation reagents, incubating the cell product with the reagents under appropriate agitation at an appropriate temperature, removing unbound reagent by centrifugation and removal of the supernatant, removing platelet content by low speed centrifugation and removal of the supernatant, passing the cells over the magnetic separation column in a magnetic field and configure the cell product in a predefined volume and solution.

### PHARMACEUTICAL COMPOSITION

The process of the invention can be used to obtain a pharmaceutical composition comprising a cell population obtainable from bone marrow or blood, wherein the cell population is depleted of TCR alpha/beta positive cells and CD45RA positive cells by 0.1 log to 7.0 log.

Preferable, the pharmaceutical composition can be obtained with the method of the invention as disclosed.

The pharmaceutical composition can be used for the reconstitution of the hematopoietic system of a human after a stem cell or bone marrow transplantation; or for the prevention and treatment of infection, mixed chimerism or cancer recurrence in the form of adoptive cell transfer.

### STEM CELL AND BONE MARROW TRANSPLANTATION

For a bone marrow transplantation, about one liter of a bone marrow-blood mixture is removed from the pelvic bone of the donor under general anesthesia.

In order to remove stem cells from the blood, the body's own hormone-like substance is administered to the donor over several days that stimulates the production of stem cells and their transfer from the bone marrow to the blood circulatory system. The methods for the pre-treatment of the donors for the removal of bone marrow or blood stem cells are state of the art and known to the skilled artisan.

The aim of the blood stem cell transplantation is to equip the recipient with a healthy stem cell population that can differentiate into blood cells. Thereby, the deficient or the pathological cells of the recipients are being replaced. In allogeneic transplantations, the tissue stems from a healthy donor. This can be an identical sibling twin, an HLA identical sibling, a non-HLA identical family member (mismatched related donor), a haploid identical donor or an unrelated HLA-compatible donor. The main target of the allogeneic transplantation is to substitute the ill or defective hematopoietic system, like for example the bone marrow of the recipient, completely by a healthy, functional hematopoietic system comprising the immune system. The stem cell transplantation can, however, also be performed with autologous, that is, the patient's own cells.

A donor of first choice is an identical sibling (Identical Sibling:IdSib) with respect to the relevant histocompatibility antigens HLA-A, B, C, DRBI and DQB1. However, such an identical sibling can only be found in ca. 30% of the cases, such that often an HLA-identical unrelated donor (matched unrelated donor, MUD) needs to be found. Since far from all histocompatibility antigens are known and only a limited number of alleles can be tested, one needs to assume a worse match with an identical unrelated donor than with a sibling donor.

A remarkable segment of the patient population remains without donor. For these patients, related donors can be used that agree with a recipient only an one haplotype of the HLA allele, that is, haplo-identical.

Transplants of unrelated donors (MUD) are used most often for hematopoietic stem cell transplantations. For un-manipulated transplants in the MUD setting, GvHD is the main complication. Severe cases of GvHD are to be regarded as life threatening and require massive therapy with immune suppressant substances for which response rates of about 40% have been described.

### TRANSPLANTATION

The actual transplantation can be divided into two phases. With the conditioning through chemo- and/or radiation therapy, the immune system of the recipient is destroyed so that the transferred or transplanted bone marrow or stem cells are not being rejected. That is to say, the recipient is being prepared for the engraftment of the transplant. The better this is achieved, the lower the risk of a non-engraftment or rejection of the transplant. Depending on the strength of the conditioning, the goal to be achieved is to destroy the remaining leukemic or malignant cells in the patient. The transplantation is performed in an intravenous manner at day 0. Until the engraftment of the transplant and the fading of the immediate toxicity, the patient remains usually in a ward suited for such a case. After the engraftment of the transplant and the waning of the immediate toxicity, a rigorous monitoring is necessary during the first three months. The intensity of the monitoring depends heavily on the type of the donor and the complications and merges into a regular life-long after care.

### INDICATIONS / USE

The cell population or pharmaceutical composition of the invention or obtained via the method of the invention may be used to treat all medical indications that require an allogeneic stem cell transplantation like inborn and acquired malignant and non-malignant diseases of hematopoietic system. The treatment may comprise allogeneic stem cell transplantation. Especially, the pharmaceutical composition obtained with the method of the invention may be used for the reconstitution of the hematopoietic system of a human after a stem cell or bone marrow transplantation. Furthermore, the pharmaceutical composition obtained with the method of the invention may be used for the prevention and treatment of infection, mixed chimerism, cancer recurrence, or immune disorders in the form of adoptive cell transfer with or without gene-modification or further cell manipulation. Further indications are malignant diseases that respond to a dose-intensification of the chemotherapy or radiation therapy.

During the treatment, immune suppressants like cyclosporine, corticosteroids, antimetabolites and monoclonal anti-lymphocytic antibodies may be used in order to control GvHD.

In a preferred embodiment of the pharmaceutical composition, the composition comprises further at least one pharmaceutically acceptable carrier or additive. Such carriers or additives are known to the person of skill in the art.

The pharmaceutical composition can be administered for treatment of cancer, such as, leukemia and other diseases, e.g. acute myeloid leukemia, acute lymphoblastic leukemia, aplastic anemia, thalassemia, inborn error (HHS) as well as against solid tumors (e.g. neuroblastoma, sarcoma etc.) for which an allogeneic transplantation is indicated or a therapeutic effect of TCR alpha/beta-, CD45-depleted cell preparations is to be expected.

Moreover, a sufficient amount of CD34+ cells need to be transferred (at least two, better more than four million per kg of body weight of the recipient) during an allogeneic transplantation in order to achieve a good reconstitution of the hematopoietic system. B cells can be retained in the transplant to preserve B-cell immunity if sufficient memory T cells are maintained after cell separation (for example more than 1 million CD45RA- T cells per kg). Alternatively, B cells that are removed from the transplant by CD19 depletion should be present in the smallest number possible or should be removed later in the recipient through, for example, the administration of an anti-CD20 antibody in vivo.

The amount to be administered to a human patient of the depleted cell population is typically between 2x10E10 to 1x10E11 lymphocytes.

While various details have been described in conjunction with the exemplary implementations outlined above, various alternatives, modifications, variations, improvements, and/or substantial equivalents, whether known or that are or may be presently unforeseen, may become apparent upon reviewing the foregoing disclosure. Accordingly, the exemplary implementations set forth above, are intended to be illustrative, not limiting.

### EXAMPLES

Antigen-specific T cells can support the immune defense after a stem cell transplantation and could protect the patient form different diseases (e.g. CMV, EBV, Influenza). By the expression of CD45RA and CD45RO T cells can be divided in memory T cells, which are CD45RO+/CD45RA- and in naive T cells which are CD45RA+/CD45RO-.

CD45RO+ memory T cells can be reactivated by repeated antigen-contact (e.g. antigens from CMV, EBV or Influenza) and produce several cytokines (e.g. IFNγ) to trigger the immune response against the infection. CD45RA depleted blood product (e.g. LP or whole blood) contains mainly CD45RO+ memory T cells, which can react against different antigens and produce INFγ.

In contrast, in TCRab-depleted cellular products, CD45RO+ memory T cells are depleted too and almost no antigen-specific T cells are expected in these cellular products.

In the method of the invention, a combination of TCRab- and CD45RA-Depletion is used as separation strategy. The target cell fraction, as an example, contains 95% of TCRab-depleted product and 5% of CD45RA depleted product.

### General description of the experiments

List of abbreviations: CMV: Cytomegalovirus, EBV: Ebstein-Barr-Virus, IFNγ: Interferon-gamma, LP: leukapheresis, MQ: MACSQuant Analyzer 10, SEB: Staphylococcal Enterotoxin B, TCRab: T cell receptor alpha beta

In the following experiments, a TCRab-Depletion and a CD45RA-Depletion was performed with one LP (½ LP for TCRab-Depletion and ½ LP for CD45RA). 95% of TCRab-depleted fraction and 5% of the CD45RA depleted fraction were mixed together and stimulated with different antigens. After 6h of incubation, the INFγ production was measured by intracellular staining and measurement at the MQ. The INFγ production of the mixture 95:5 was compared to the unseparated LP and to the TCRab-depleted fraction.

### Step-by-step description of the experiments

A) Split of leukapheresis sample
   - One half is used for LP-TCRab-Depletion with CliniMACS Prodigy (Miltenyi Biotec GmbH)
   - One half is used for CD45RA Depletion with CliniMACS Prodigy (Miltenyi Biotec GmbH)
B) After Depletion TCRab depleted fraction and CD45RA depleted fraction is mixed in a cell ratio of 95:5
C) The following fractions are used for the Rapid Cytokine Assay (detection of antigen specific T cells), performed according to the datasheet Rapid Cytokine Inspector (CD4/CD8 T Cell) Kit, 130-097-343 (Miltenyi Biotec GmbH)
   - Unseparated leukapheresis
   - TCRab depleted fraction
   - Mixture 95:5 (TCRab depleted : CD45RA depleted fraction)
D) All fractions are stimulated with the following antigens according to the datasheet of the respective stimulation agent:
   - CEF-Pool → datasheet 130-098-426 (PepTivator@ CEF MHC Class I Plus) (Miltenyi Biotec GmbH)
   - CMV (pp56 and IE-1 Peptivator) → datasheet 130-093-493 (Miltenyi Biotec GmbH) (PepTivator@ CMV IE-1) and 130-093-438 (PepTivator@ CMV pp65) (Miltenyi Biotec GmbH)
   - EBV-Consensus-Pool → datasheet 130-099-764 (PepTivator@ EBV Consensus) (Miltenyi Biotec GmbH)
   - SEB (positive control)
   - No stimulation (negative control)
E) Stimulation of cell for 6h at 37°C, after 2h Brefeldin A is added (Block of exocytosis)
F) Intracellular staining with anti-IFNy-PE and Rapid Cytokine Inspector, performed according to the datasheet Rapid Cytokine Inspector (CD4/CD8 T Cell) Kit, 130-097-343 (Miltenyi Biotec GmbH)and Rapid Cytokine Inspector Anti-Cytokine Antibodies, 130-097-600 (Miltenyi Biotec GmbH)
G) Measuring of stained cells at the MACSQuant Analyzer 10 (Miltenyi Biotec GmbH)

### Results of the experiments

The higher the frequency of IFNγ+ T cells (IFNγ-Production), the higher the reaction of antigen-specific cells and the higher the positive effect for the stem cell transplantation. This effect is expected for the mixture 95:5 but not for the TCRab-depleted target cell fraction.

The mixture 95:5 (TCRab depleted : CD45RA depleted fraction) shows a production of IFNγ after stimulation with CEF-Pool and CMV compared to the TCRab depleted fraction. This show that the mixture 95:5 contains antigen-specific T cells against CMV and peptides from CEF-Pool (CMV, EBV, Influenza), which is an advantage compared to the TCRab-depleted fraction.

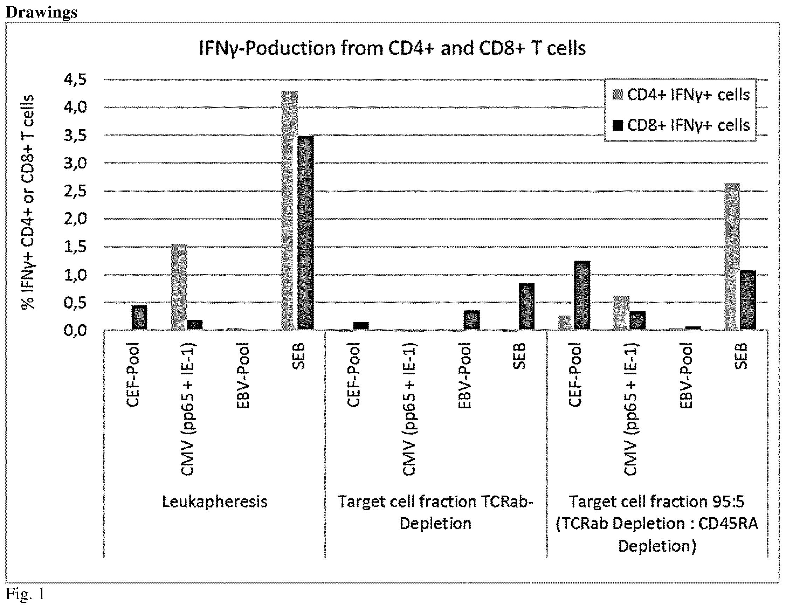

Fig. 1 shows the IFNγ-Production by CD4+ and CD8+ T cells for the leukapheresis, TCRab depleted fraction and mixture 95:5 (TCRab depleted : CD45RA depleted) minus the negative control for each sample . The positive effect of IFNγ-production can be seen from the column "mixture 95:5 (TCRab depleted : CD45RA depleted)" as compared to the TCRab depleted fraction.

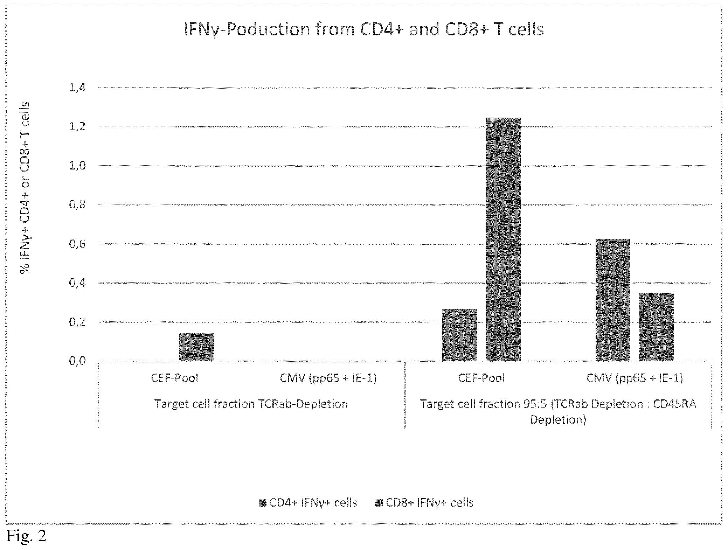

Fig. 2 shows IFNγ-Production by CD4+ and CD8+ T cells for TCRab depleted fraction and mixture 95:5 (TCRab depleted : CD45RA depleted) minus the negative control for each sample. The cells from the TCRab depleted fraction shown no reaction, i.e. no IFNγ-Production, whereas cells obtained by the method of the invention show the positive effect of IFNγ-Production.

The antigen-specific T cells obtained with the method of the invention can support the immune defense after a stem cell transplantation and could protect the patient form different diseases (e.g. CMV, EBV, Influenza).

### Reduction of alloreactivity

Non-processed apheresis products contain alloreactive T cells, possibly resulting in severe side effects after infusion to a patient. Alloreactivity is removed by TCRalpha/beta depletion. The combination of TCRalpha/beta depleted and CD45RA depleted products could contain alloreactive cells if they are included in the CD45RA depleted product.

Procedure: Peripheral blood mononuclear cells were separated into CD45RA positive and CD45RA negative cells by magnetic cell sorting. Gamma irradiated third party PBMCs were used as stimulator cells to activate CD45RA positive or negative responder cells in a co-culture system. Proliferative response of CD45RA positive and negative cells was assessed by BrdU count. Activation state was assessed by quantification of Interferon-gamma in the cell culture supernatants. Three independent experiments were performed.

Results: CD45RA positive cells had a significantly higher BrdU count compared to CD45RA negative cells (200000 vs. 40000), indicating for active proliferation in CD45RA positive but not CD45RA negative cell upon interaction with allogeneic cells. Interferon gamma was detected in the supernatant of CD45RA positive but not CD45RA negative cells upon interaction with allogeneic cells.

Conclusion: Addition of CD45RA depleted cells to a TCRalpha/beta depleted stem cell product does not add unwanted alloreactive potential to the stem cell product.

### Manufacturing of cell product / pharmaceutical composition

On CliniMACS plus: Set separation program DEPLETION 3.1 and use CliniMACS Depletion Tubing Set. CD45RA labelled cells (5%) were added to the re-application bag shortly before sensitive depletion of a DEPLETION 3.1 / Depletion Tubing Set run with a TCRab labelled product was completed.

### Composition before Depletion:

24.5% TCRab+/CD45RA+ cells (of 1.12E10 = 2.74E9)

### Composition after depletion:

0.03% TCRab+/CD45RA+ cells (of 3.33E9 = 1E6), 3.43 log depletion of TCRab+/CD45RA+ cells

## Claims

1. Method for the preparation of a cell population from a sample originating from bone marrow or blood, comprising the steps a) dividing the sample into a first fraction containing 50 to 99 % of the cells of the sample and a second fraction containing 50 to 1 % of the cells of the sample, b) labeling the cells of the first fraction with a first marker against TCR alpha/beta, c) labeling the cells of the second fraction with a second marker against CD45RA, d) removing the labeled cells from the first and second fraction and combining the remaining cells to a cell population.

2. Method according to claim 1, **characterized in that** the first marker comprises an antibody or antigen-binding fragment against TCR alpha/beta and a detection moiety.

3. Method according to claim 1, **characterized in that** the second marker comprises an antibody or antigen-binding fragment against CD45RA and a detection moiety.

4. Method according to any of the claim 1 to 3, **characterized in** labeling cells of the first fraction and/or the second fraction and/or the cell population with a third marker against CD19 and removing the labeled cells.

5. Method according to claim 4, **characterized in that** the third marker comprises an antibody or antigen-binding fragment against CD19 and a detection moiety.

6. Method according to any of the claim 1 to 5, **characterized in that** the detection moiety of the first and/or second and/or third marker is a fluorescence dye, a magnetic particle or a radioactive label.

7. Method according to any of the claims 1 to 6, **characterized in that** in step d), first and second fraction are combined and the labeled cells are removed from the combined fraction.

8. Method according to any of the claims 1 to 6, **characterized in that** in step d), labeled cells are removed from the first and second fraction separately and the remaining cells of each fraction are combined.

## Patentansprüche

1. Verfahren zur Herstellung einer Zellpopulation aus einer aus Knochenmark oder Blut stammenden Probe, umfassend die Schritte zum a) Aufteilen der Probe in eine erste Fraktion, die 50 bis 99 % der Zellen der Probe enthält, und eine zweite Fraktion, die 50 bis 1 % der Zellen der Probe enthält, b) Markieren der Zellen der ersten Fraktion mit einem ersten Marker gegen TCR alpha/beta, c) Markieren der Zellen der zweiten Fraktion mit einem zweiten Marker gegen CD45RA, d) Entfernen der markierten Zellen aus der ersten und zweiten Fraktion und Kombinieren der verbleibenden Zellen zu einer Zellpopulation.

2. Verfahren nach Anspruch 1, **dadurch gekennzeichnet, dass** der erste Marker einen Antikörper oder ein Antigen-bindendes Fragment gegen TCR alpha/beta und eine Nachweiseinheit umfasst.

3. Verfahren nach Anspruch 1, **dadurch gekennzeichnet, dass** der zweite Marker einen Antikörper oder ein Antigen-bindendes Fragment gegen CD45RA und eine Nachweiseinheit umfasst.

4. Verfahren nach einem der Ansprüche 1 bis 3, **gekennzeichnet durch** das Markieren von Zellen der ersten Fraktion und/oder der zweiten Fraktion und/oder der Zellpopulation mit einem dritten Marker gegen CD19 und Entfernen der markierten Zellen.

5. Verfahren nach Anspruch 4, **dadurch gekennzeichnet, dass** der dritte Marker einen Antikörper oder ein Antigen-bindendes Fragment gegen CD19 und eine Nachweiseinheit umfasst.

6. Verfahren nach einem der Ansprüche 1 bis 5, **dadurch gekennzeichnet, dass** die Nachweiseinheit des ersten und/oder zweiten und/oder dritten Markers ein Fluoreszenzfarbstoff, ein magnetisches Teilchen oder eine radioaktive Markierung ist.

7. Verfahren nach einem der Ansprüche 1 bis 6, **dadurch gekennzeichnet, dass** in Schritt d) die erste und zweite Fraktion kombiniert werden und die markierten Zellen aus der kombinierten Fraktion entfernt werden.

8. Verfahren nach einem der Ansprüche 1 bis 6, **dadurch gekennzeichnet, dass** in Schritt d) die markierten Zellen getrennt von der ersten und zweiten Fraktion entfernt werden und die verbleibenden Zellen jeder Fraktion kombiniert werden.

## Revendications

1. Procédé de préparation d'une population de cellules à partir d'un échantillon provenant de moelle osseuse ou de sang, comprenant les étapes de a) diviser l'échantillon en une première fraction contenant 50 à 99 % des cellules de l'échantillon et une seconde fraction contenant 50 à 1 % des cellules de l'échantillon, b) marquer les cellules de la première fraction avec un premier marqueur contre le TCR alpha/béta, c) marquer les cellules de la seconde fraction avec un second marqueur contre le CD45RA, d) enlever les cellules marquées des première et seconde fractions et combiner les cellules restantes en une population de cellules.

2. Procédé selon la revendication 1, **caractérisé en ce que** le premier marqueur comprend un anticorps ou un fragment de liaison à un antigène contre le TCR alpha/béta et une fraction de détection.

3. Procédé selon la revendication 1, **caractérisé en ce que** le second marqueur comprend un anticorps ou un fragment de liaison à un antigène contre le CD45RA et une fraction de détection.

4. Procédé selon l'une quelconque des revendications 1 à 3, **caractérisé en ce qu'**un marquage des cellules de la première fraction et/ou de la seconde fraction et/ou de la population de cellules avec un troisième marqueur contre le CD19 et l'enlèvement des cellules marquées.

5. Procédé selon la revendication 4, **caractérisé en ce que** le troisième marqueur comprend un anticorps ou un fragment de liaison à un antigène contre le CD19 et une fraction de détection.

6. Procédé selon l'une quelconque des revendications 1 à 5, **caractérisé en ce que** la fraction de détection du premier et/ou du deuxième et/ou du troisième marqueur est un colorant de fluorescence, une particule magnétique ou un marqueur radioactif.

7. Procédé selon l'une quelconque des revendications 1 à 6, **caractérisé en ce que** dans l'étape d), la première et la deuxième fraction sont combinées et les cellules marquées sont enlevées de la fraction combinée.

8. Procédé selon l'une quelconque des revendications 1 à 6, **caractérisé en ce que** dans l'étape d), les cellules marquées sont enlevées des première et deuxième fractions séparément et les cellules restantes de chaque fraction sont combinées.


### SEQUENCE LISTING

<110> Miltenyi Biotec GmbH
<120> CELL COMPOSITION DEPLETED FROM TCRab and CD45RA POSITIVE CELLS
<130> MIL_104
<160> 11
<170> BiSSAP 1.3.6
<210> 1
   <211> 142
   <212> PRT
   <213> Homo sapiens
<400> 1
   Pro Asn Ile Gln Asn Pro Asp Pro Ala Val Tyr Gln Leu Arg Asp Ser
   1               5                   10                  15
   Lys Ser Ser Asp Lys Ser Val Cys Leu Phe Thr Asp Phe Asp Ser Gln
               20                  25                  30
   Thr Asn Val Ser Gln Ser Lys Asp Ser Asp Val Tyr Ile Thr Asp Lys
           35                  40                  45
   Thr Val Leu Asp Met Arg Ser Met Asp Phe Lys Ser Asn Ser Ala Val
       50                  55                  60
   Ala Trp Ser Asn Lys Ser Asp Phe Ala Cys Ala Asn Ala Phe Asn Asn
   65                  70                  75                  80
   Ser Ile Ile Pro Glu Asp Thr Phe Phe Pro Ser Pro Glu Ser Ser Cys
                   85                  90                  95
   Asp Val Lys Leu Val Glu Lys Ser Phe Glu Thr Asp Thr Asn Leu Asn
               100                 105                 110
   Phe Gln Asn Leu Ser Val Ile Gly Phe Arg Ile Leu Leu Leu Lys Val
           115                 120                 125
   Ala Gly Phe Asn Leu Leu Met Thr Leu Arg Leu Trp Ser Ser
       130                 135                 140
<210> 2
   <211> 426
   <212> DNA
   <213> Homo sapiens
<400> 2
   ccaaatatcc agaaccctga ccctgccgtg taccagctga gagactctaa atccagtgac      60
   aagtctgtct gcctattcac cgattttgat tctcaaacaa atgtgtcaca aagtaaggat     120
   tctgatgtgt atatcacaga caaaactgtg ctagacatga ggtctatgga cttcaagagc     180
   aacagtgctg tggcctggag caacaaatct gactttgcat gtgcaaacgc cttcaacaac     240
   agcattattc cagaagacac cttcttcccc agcccagaaa gttcctgtga tgtcaagctg     300
   gtcgagaaaa gctttgaaac agatacgaac ctaaactttc aaaacctgtc agtgattggg     360
   ttccgaatcc tcctcctgaa agtggccggg tttaatctgc tcatgacgct gcggctgtgg     420
   tccagc                                                                426
<210> 3
   <211> 3430
   <212> DNA
   <213> Homo sapiens
<400> 3
   ttcccgtata aagcatgaga ccgtgacttg ccagccccac agagccccgc ccttgtccat       60
   cactggcatc tggactccag cctgggttgg ggcaaagagg gaaatgagat catgtcctaa      120
   ccctgatcct cttgtcccac agatatccag aaccctgacc ctgccgtgta ccagctgaga      180
   gactctaaat ccagtgacaa gtctgtctgc ctattcaccg attttgattc tcaaacaaat      240
   gtgtcacaaa gtaaggattc tgatgtgtat atcacagaca aaactgtgct agacatgagg      300
   tctatggact tcaagagcaa cagtgctgtg gcctggagca acaaatctga ctttgcatgt      360
   gcaaacgcct tcaacaacag cattattcca gaagacacct tcttccccag cccaggtaag      420
   ggcagctttg gtgccttcgc aggctgtttc cttgcttcag gaatggccag gttctgccca      480
   gagctctggt caatgatgtc taaaactcct ctgattggtg gtctcggcct tatccattgc      540
   caccaaaacc ctctttttac taagaaacag tgagccttgt tctggcagtc cagagaatga      600
   cacgggaaaa aagcagatga agagaaggtg gcaggagagg gcacgtggcc cagcctcagt      660
   ctctccaact gagttcctgc ctgcctgcct ttgctcagac tgtttgcccc ttactgctct      720
   tctaggcctc attctaagcc ccttctccaa gttgcctctc cttatttctc cctgtctgcc      780
   aaaaaatctt tcccagctca ctaagtcagt ctcacgcagt cactcattaa cccaccaatc      840
   actgattgtg ccggcacatg aatgcaccag gtgttgaagt ggaggaatta aaaagtcaga      900
   tgaggggtgt gcccagagga agcaccattc tagttggggg agcccatctg tcagctggga      960
   aaagtccaaa taacttcaga ttggaatgtg ttttaactca gggttgagaa aacagccacc     1020
   ttcaggacaa aagtcaggga agggctctct gaagaaatgc tacttgaaga taccagccct     1080
   accaagggca gggagaggac cctatagagg cctgggacag gagctcaatg agaaaggaga     1140
   agagcagcag gcatgagttg aatgaaggag gcagggccgg gtcacagggc cttctaggcc     1200
   atgagagggt agacagtatt ctaagtacgc cagaaagctg ttgatcggct tcaagcaggg     1260
   aagggacacc taatttgctt ttcttttctt tttttttttt tttttttttt tttttttgag      1320
   atggagtttt gctcttgttg cccaggctgg agtgcaatgg tgcatcttgg ctcactacaa     1380
   gcctctgcct cccaggttca agtgattctc ctgcctcagc ctcccaagta gctgggatta     1440
   caggcaccca ccaccatgcc cggctaattt tttgtatttt tagtagagac agggtttcac     1500
   tatgttggcc aggctggtct cgaactcctg acctcaggtg atccacccgc ttcagcctcc     1560
   caaagtgctg ggattacagg cgtgagccac cacacccggc ctgcttttct taaagatcaa     1620
   tctgagtgct gtacggagag tgggttgtaa gccaagagta gaagcagaaa gggagcagtt     1680
   gcagcagaga gatgatggag gcctgggcac ggtggtggca gggaggtaac caacaccatt     1740
   caggtttcaa aggtagaacc atgcagggat gagaaagcaa agaggggatc aaggaaggca 1800
   gctggatttt ggcctgagca gctgagtcaa tgatagtgcc gtttactaag aagaaaccaa 1860
   ggaaaaaatt tggggtgcag ggatcaaaac tttttggaac atatgaaagt acgtgtttat 1920
   actctttatg gcccttgtca ctatgtatgc ctcgctgcct cattggactc tagaatgaag 1980
   ccaggcagag cagggtctat gtgtgatggc acatgtggcc agggtcatgc agacatgtac 2040
   tttgtacaaa cagtgtatat tgagtaaata gaaatggtgt ccaggagccg aggtatcgtc 2100
   ctgccagggc caggggctct ccctagcagg tgctcatatg ctgtaagttc cctccagatc 2160
   tctccacaag gaggcatgga aaggctgtag ttgttcacct gcccaagaac taggaggtct 2220
   ggggtgggag agtcagcctg ctctggatgc tgaaagaatg tctgtttttc cttttagaaa 2280
   gttcctgtga tgtcaagctg gtcgagaaaa gctttgaaac aggtaagaca ggggtctagc 2340
   ctgggtttgc acaggattgc cgaagtgatg aacccgcaat aaccctgcct ggatgaggga 2400
   gtgggaagaa attagtagat gtgggaatga atgatgagga atggaaacag cggttcaaga 2460
   cctgcccaga gctgggtggg gtctctcctg aatccctctc accgtctctg actttccgtt 2520
   ctaagcactt tgaggatgag tttctagctt caatagacca aggactctct cctaggcctc 2580
   tgtattcctt tcaacagctc cactgtcaag agagccagag agagcttctg ggtggcccag 2640
   ctgtgaaatt tctgagtccc ttagggatag ccctaaacga accagatcat cctgaggaca 2700
   gccaagaggt tttgccttct ttcaagacaa gcaacagtac tcacataggc tgtgggcaat 2760
   ggtcctgtct ctcaagaatc ccctgccact cctcacaccc accctgggcc catattcatt 2820
   tccatttgag ttgttcttat tgagtcatcc ttcctgtggc agcggaactc actaaggggc 2880
   ccatctggac ccgaggtatt gtgaagataa attctgagca cctaccccat ccccagaagg 2940
   gctcagaaat aaaataagag ccaagtctag tcggtgtttc ctgtcttgaa acacaatact 3000
   gttggccctg gaagaatgca cagaatctgt ttgtaagggg atatgcacag aagctgcaag 3060
   ggacaggagg tgcaggagct gcaggcctcc cccacccagc ctgctctgcc ttggggaaaa 3120
   ccgtgggtgt gtcctgcagg ccatgcaggc ctgggacatg caagcccata accgctgtgg 3180
   cctcttggtt ttacagatac gaacctaaac tttcaaaacc tgtcagtgat tgggttccga 3240
   atcctcctcc tgaaagtggc cgggtttaat ctgctcatga cgctgcggct gtggtccagc 3300
   tgaggtgagg ggccttgaag ctgggagtgg ggtttaggga cgcgggtctc tgcgtgcatc 3360
   ctaagctctg agagcaaacc tccctgcagg gtcttgcttt taagtccaaa gcctgagccc 3420
   accaaactct 3430
<210> 4
   <211> 177
   <212> PRT
   <213> Homo sapiens
<400> 4
   Glu Asp Leu Asn Lys Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Phe Pro Asp His Val Glu Leu Ser Trp Trp Val Asn
           35                  40                  45
   Gly Lys Glu Val His Ser Gly Val Ser Thr Asp Pro Gln Pro Leu Lys
       50                  55                  60
   Glu Gln Pro Ala Leu Asn Asp Ser Arg Tyr Cys Leu Ser Ser Arg Leu
   65                  70                  75                  80
   Arg Val Ser Ala Thr Phe Trp Gln Asn Pro Arg Asn His Phe Arg Cys
                   85                  90                  95
   Gln Val Gln Phe Tyr Gly Leu Ser Glu Asn Asp Glu Trp Thr Gln Asp
               100                 105                 110
   Arg Ala Lys Pro Val Thr Gln Ile Val Ser Ala Glu Ala Trp Gly Arg
           115                 120                 125
   Ala Asp Cys Gly Phe Thr Ser Val Ser Tyr Gln Gln Gly Val Leu Ser
       130                 135                 140
   Ala Thr Ile Leu Tyr Glu Ile Leu Leu Gly Lys Ala Thr Leu Tyr Ala
   145                 150                 155                 160
   Val Leu Val Ser Ala Leu Val Leu Met Ala Met Val Lys Arg Lys Asp
                   165                 170                 175
   Phe
<210> 5
   <211> 531
   <212> DNA
   <213> Homo sapiens
<400> 5
   gaggacctga acaaggtgtt cccacccgag gtcgctgtgt ttgagccatc agaagcagag      60
   atctcccaca cccaaaaggc cacactggtg tgcctggcca caggcttctt ccccgaccac     120
   gtggagctga gctggtgggt gaatgggaag gaggtgcaca gtggggtcag cacagacccg     180
   cagcccctca aggagcagcc cgccctcaat gactccagat actgcctgag cagccgcctg     240
   agggtctcgg ccaccttctg gcagaacccc cgcaaccact tccgctgtca agtccagttc     300
   tacgggctct cggagaatga cgagtggacc caggataggg ccaaacccgt cacccagatc     360
   gtcagcgccg aggcctgggg tagagcagac tgtggcttta cctcggtgtc ctaccagcaa     420
   ggggtcctgt ctgccaccat cctctatgag atcctgctag ggaaggccac cctgtatgct     480
   gtgctggtca gcgcccttgt gttgatggcc atggtcaaga gaaaggattt c              531
<210> 6
   <211> 2042
   <212> DNA
   <213> Homo sapiens
<400> 6
   tgcatcctag ggacagcata gaaaggaggg gcaaagtgga gagagagcaa cagacactgg 60
   gatggtgacc ccaaaacaat gagggcctag aatgacatag ttgtgcttca ttacggccca 120
   ttcccagggc tctctctcac acacacagag cccctaccag aaccagacag ctctcagagc 180
   aaccctggct ccaacccctc ttccctttcc agaggacctg aacaaggtgt tcccacccga 240
   ggtcgctgtg tttgagccat cagaagcaga gatctcccac acccaaaagg ccacactggt 300
   gtgcctggcc acaggcttct tccccgacca cgtggagctg agctggtggg tgaatgggaa 360
   ggaggtgcac agtggggtca gcacggaccc gcagcccctc aaggagcagc ccgccctcaa 420
   tgactccaga tactgcctga gcagccgcct gagggtctcg gccaccttct ggcagaaccc 480
   ccgcaaccac ttccgctgtc aagtccagtt ctacgggctc tcggagaatg acgagtggac 540
   ccaggatagg gccaaacccg tcacccagat cgtcagcgcc gaggcctggg gtagagcagg 600
   tgagtggggc ctggggagat gcctggagga gattaggtga gaccagctac cagggaaaat 660
   ggaaagatcc aggtagcaga caagactaga tccaaaaaga aaggaaccag cgcacaccat 720
   gaaggagaat tgggcacctg tggttcattc ttctcccaga ttctcagccc aacagagcca 780
   agcagctggg tcccctttct atgtggcctg tgtaactctc atctgggtgg tgccccccat 840
   ccccctcagt gctgccacat gccatggatt gcaaggacaa tgtggctgac atctgcatgg 900
   cagaagaaag gaggtgctgg gctgtcagag gaagctggtc tgggcctggg agtctgtgcc 960
   aactgcaaat ctgactttac ttttaattgc ctatgaaaat aaggtctctc atttattttc 1020
   ctctccctgc tttctttcag actgtggctt tacctcgggt aagtaagccc ttccttttcc 1080
   tctccctctc tcatggttct tgacctagaa ccaaggcatg aagaactcac agacactgga 1140
   gggtggaggg tgggagagac cagagctacc tgtgcacagg tacccacctg tccttcctcc 1200
   gtgccaacag tgtcctacca gcaaggggtc ctgtctgcca ccatcctcta tgagatcctg 1260
   ctagggaagg ccaccctgta tgctgtgctg gtcagcgccc ttgtgttgat ggccatggta 1320
   agcaggaggg caggatgggg ccagcaggct ggaggtgaca cactgacacc aagcacccag 1380
   aagtatagag tccctgccag gattggagct gggcagtagg gagggaagag atttcattca 1440
   ggtgcctcag aagataactt gcacctctgt aggatcacag tggaagggtc atgctgggaa 1500
   ggagaagctg gagtcaccag aaaacccaat ggatgttgtg atgagcctta ctatttgtgt 1560
   ggtcaatggg ccctactact ttctctcaat cctcacaact cctggctctt aataaccccc 1620
   aaaactttct cttctgcagg tcaagagaaa ggatttctga aggcagccct ggaagtggag 1680
   ttaggagctt ctaacccgtc atggtttcaa tacacattct tcttttgcca gcgcttctga 1740
   agagctgctc tcacctctct gcatcccaat agatatcccc ctatgtgcat gcacacctgc 1800
   acactcacgg ctgaaatctc cctaacccag ggggacctta gcatgcctaa gtgactaaac 1860
   caataaaaat gttctggtct ggcctgactc tgacttgtga atgtctggat agctccttgg 1920
   ctgtctctga actccctgtg actctcccca ttcagtcagg atagaaacaa gaggtattca    1980
   aggaaaatgc agactcttca cgtaagaggg atgaggggcc caccttgaga tcaatagcag    2040
   aa                                                                   2042
<210> 7
   <211> 179
   <212> PRT
   <213> Homo sapiens
<400> 7
   Glu Asp Leu Lys Asn Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Tyr Pro Asp His Val Glu Leu Ser Trp Trp Val Asn
           35                  40                  45
   Gly Lys Glu Val His Ser Gly Val Ser Thr Asp Pro Gln Pro Leu Lys
       50                  55                  60
   Glu Gln Pro Ala Leu Asn Asp Ser Arg Tyr Cys Leu Ser Ser Arg Leu
   65                  70                  75                  80
   Arg Val Ser Ala Thr Phe Trp Gln Asn Pro Arg Asn His Phe Arg Cys
                   85                  90                  95
   Gln Val Gln Phe Tyr Gly Leu Ser Glu Asn Asp Glu Trp Thr Gln Asp
               100                 105                 110
   Arg Ala Lys Pro Val Thr Gln Ile Val Ser Ala Glu Ala Trp Gly Arg
           115                 120                 125
   Ala Asp Cys Gly Phe Thr Ser Glu Ser Tyr Gln Gln Gly Val Leu Ser
       130                 135                 140
   Ala Thr Ile Leu Tyr Glu Ile Leu Leu Gly Lys Ala Thr Leu Tyr Ala
   145                 150                 155                 160
   Val Leu Val Ser Ala Leu Val Leu Met Ala Met Val Lys Arg Lys Asp
                   165                 170                 175
   Ser Arg Gly
<210> 8
   <211> 178
   <212> PRT
   <213> Homo sapiens
<400> 8
   Asp Leu Lys Asn Val Phe Pro Pro Glu Val Ala Val Phe Glu Pro Ser
   1               5                   10                  15
   Glu Ala Glu Ile Ser His Thr Gln Lys Ala Thr Leu Val Cys Leu Ala
               20                  25                  30
   Thr Gly Phe Tyr Pro Asp His Val Glu Leu Ser Trp Trp Val Asn Gly
           35                  40                  45
   Lys Glu Val His Ser Gly Val Ser Thr Asp Pro Gln Pro Leu Lys Glu
       50                  55                  60
   Gln Pro Ala Leu Asn Asp Ser Arg Tyr Cys Leu Ser Ser Arg Leu Arg
   65                  70                  75                  80
   Val Ser Ala Thr Phe Trp Gln Asn Pro Arg Asn His Phe Arg Cys Gln
                   85                  90                  95
   Val Gln Phe Tyr Gly Leu Ser Glu Asn Asp Glu Trp Thr Gln Asp Arg
               100                 105                 110
   Ala Lys Pro Val Thr Gln Ile Val Ser Ala Glu Ala Trp Gly Arg Ala
           115                 120                 125
   Asp Cys Gly Phe Thr Ser Glu Ser Tyr Gln Gln Gly Val Leu Ser Ala
       130                 135                 140
   Thr Ile Leu Tyr Glu Ile Leu Leu Gly Lys Ala Thr Leu Tyr Ala Val
   145                 150                 155                 160
   Leu Val Ser Ala Leu Val Leu Met Ala Met Val Lys Arg Lys Asp Ser
                   165                 170                 175
   Arg Gly
<210> 9
   <211> 537
   <212> DNA
   <213> Homo sapiens
<400> 9
   gaggacctga aaaacgtgtt cccacccgag gtcgctgtgt ttgagccatc agaagcagag      60
   atctcccaca cccaaaaggc cacactggtg tgcctggcca caggcttcta ccccgaccac     120
   gtggagctga gctggtgggt gaatgggaag gaggtgcaca gtggggtcag cacagacccg     180
   cagcccctca aggagcagcc cgccctcaat gactccagat actgcctgag cagccgcctg     240
   agggtctcgg ccaccttctg gcagaacccc cgcaaccact tccgctgtca agtccagttc     300
   tacgggctct cggagaatga cgagtggacc caggataggg ccaaacctgt cacccagatc     360
   gtcagcgccg aggcctgggg tagagcagac tgtggcttca cctccgagtc ttaccagcaa     420
   ggggtcctgt ctgccaccat cctctatgag atcttgctag ggaaggccac cttgtatgcc     480
   gtgctggtca gtgccctcgt gctgatggcc atggtcaaga gaaaggattc cagaggc        537
<210> 10
   <211> 534
   <212> DNA
   <213> Homo sapiens
<400> 10
   gacctgaaaa acgtgttccc acccgaggtc gctgtgtttg agccatcaga agcagagatc      60
   tcccacaccc aaaaggccac actggtgtgc ctggccacag gcttctaccc cgaccacgtg     120
   gagctgagct ggtgggtgaa tgggaaggag gtgcacagtg gggtcagcac agacccgcag      180
   cccctcaagg agcagcccgc cctcaatgac tccagatact gcctgagcag ccgcctgagg     240
   gtctcggcca ccttctggca gaacccccgc aaccacttcc gctgtcaagt ccagttctac     300
   gggctctcgg agaatgacga gtggacccag gatagggcca aacctgtcac ccagatcgtc     360
   agcgccgagg cctggggtag agcagactgt ggcttcacct ccgagtctta ccagcaaggg     420
   gtcctgtctg ccaccatcct ctatgagatc ttgctaggga aggccacctt gtatgccgtg     480
   ctggtcagtg ccctcgtgct gatggccatg gtcaagagaa aggattccag aggc           534
<210> 11
   <211> 2008
   <212> DNA
   <213> Homo sapiens
<400> 11
   atggcgtagt ccccaaagaa cgaggaccta gtaacataat tgtgcttcat tatggtcctt      60
   tcccggcctt ctctctcaca catacacaga gcccctacca ggaccagaca gctctcagag     120
   caaccctagc cccattacct cttccctttc cagaggacct gaaaaacgtg ttcccacccg     180
   aggtcgctgt gtttgagcca tcagaagcag agatctccca cacccaaaag gccacactgg     240
   tgtgcctggc cacaggcttc taccccgacc acgtggagct gagctggtgg gtgaatggga     300
   aggaggtgca cagtggggtc agcacagacc cgcagcccct caaggagcag cccgccctca     360
   atgactccag atactgcctg agcagccgcc tgagggtctc ggccaccttc tggcagaacc     420
   cccgcaacca cttccgctgt caagtccagt tctacgggct ctcggagaat gacgagtgga     480
   cccaggatag ggccaaacct gtcacccaga tcgtcagcgc cgaggcctgg ggtagagcag     540
   gtgagtgggg cctggggaga tgcctggagg agattaggtg agaccagcta ccagggaaaa     600
   tggaaagatc caggtagcgg acaagactag atccagaaga aagccagagt ggacaaggtg     660
   ggatgatcaa ggttcacagg gtcagcaaag cacggtgtgc acttccccca ccaagaagca     720
   tagaggctga atggagcacc tcaagctcat tcttccttca gatcctgaca ccttagagct     780
   aagctttcaa gtctccctga ggaccagcca tacagctcag catctgagtg gtgtgcatcc     840
   cattctcttc tggggtcctg gtttcctaag atcatagtga ccacttcgct ggcactggag     900
   cagcatgagg gagacagaac cagggctatc aaaggaggct gactttgtac tatctgatat     960
   gcatgtgttt gtggcctgtg agtctgtgat gtaaggctca atgtccttac aaagcagcat    1020
   tctctcatcc atttttcttc ccctgttttc tttcagactg tggcttcacc tccggtaagt    1080
   gagtctctcc tttttctctc tatctttcgc cgtctctgct ctcgaaccag ggcatggaga    1140
   atccacggac acaggggcgt gagggaggcc agagccacct gtgcacaggt acctacatgc    1200
   tctgttcttg tcaacagagt cttaccagca aggggtcctg tctgccacca tcctctatga    1260
   gatcttgcta gggaaggcca ccttgtatgc cgtgctggtc agtgccctcg tgctgatggc    1320
   catggtaagg aggagggtgg gatagggcag atgatggggg caggggatgg aacatcacac    1380
   atgggcataa aggaatctca gagccagagc acagcctaat atatcctatc acctcaatga    1440
   aaccataatg aagccagact ggggagaaaa tgcagggaat atcacagaat gcatcatggg    1500
   aggatggaga caaccagcga gccctactca aattaggcct cagagcccgc ctcccctgcc    1560
   ctactcctgc tgtgccatag cccctgaaac cctgaaaatg ttctctcttc cacaggtcaa    1620
   gagaaaggat tccagaggct agctccaaaa ccatcccagg tcattcttca tcctcaccca    1680
   ggattctcct gtacctgctc ccaatctgtg ttcctaaaag tgattctcac tctgcttctc    1740
   atctcctact tacatgaata cttctctctt ttttctgttt ccctgaagat tgagctccca    1800
   acccccaagt acgaaatagg ctaaaccaat aaaaaattgt gtgttgggcc tggttgcatt    1860
   tcaggagtgt ctgtggagtt ctgctcatca ctgacctatc ttctgattta gggaaagcag    1920
   cattcgcttg gacatctgaa gtgacagccc tctttctctc cacccaatgc tgctttctcc    1980
   tgttcatcct gatggaagtc tcaacaca                                       2008